# EUROPEAN PATENT SPECIFICATION

(11) **EP 1 613 769 B1**
(45) Date of publication and mention of the grant of the patent: **18.06.2008**
(21) Application number: 04721522.3
(22) Date of filing: 18.03.2004
(51) Int. Cl.: C12Q 1/68

(54) **INSULIN-INDUCED GENE AS THERAPEUTIC TARGET IN DIABETES**
INSULIN-INDUZIERTES GEN ALS THERAPEUTISCHES ZIEL BEI DIABETES
GENE INDUIT PAR L'INSULINE UTILISE COMME CIBLE THERAPEUTIQUE DANS LE DIABETE

(30) Priority: 17.04.2003 FR 0304835
(43) Date of publication of application: 11.01.2006
(73) Proprietor: Merck Patent GmbH, 64293 Darmstadt (DE); CENTRE NATIONAL DE LA RECHERCHE SCIENTIFIQUE (CNRS), 75016 Paris (FR)
(72) Inventor: ASFARI, Maryam, F-91600 Savigny sur Orge (FR); COFFY, Sandrine, F-91380 Chilly Mazarin (FR)
(86) International application number: PCT/EP2004/002809
(87) International publication number: WO 2004/092411

(56) References cited:
- WO-A-99/00123
- WO-A-02/059621
- DATABASE EMBL [Online] Hum. spliced transcr. det. olig. SEQ ID NO:13780, 15 July 2002 (2002-07-15) retrieved from EBI Database accession no. ABN41032 XP002286307 & WO 02/10449 A (COMPUGEN INC.) 7 February 2002 (2002-02-07)
- DATABASE EMBL [Online] seqLPD#608pr51, 17 October 2001 (2001-10-17) retrieved from EBI Database accession no. AAH43150 XP002286308 -& WO 01/57189 A (QUARK BIOTECH INC.) 9 August 2001 (2001-08-09)
- CHARPENTIER APRIL H ET AL: "Effects of estrogen on global gene expression: Identification of novel targets of estrogen action" CANCER RESEARCH, vol. 60, no. 21, 1 November 2000 (2000-11-01), pages 5977-5983, XP002263160 ISSN: 0008-5472 cited in the application
- DATABASE MEDLINE [Online] US NATIONAL LIBRARY OF MEDICINE (NLM), BETHESDA, MD, US; October 2002 (2002-10) MCMANUS MICHAEL T ET AL: "Gene silencing in mammals by small interfering RNAs." Database accession no. NLM12360232 XP002286309 & NATURE REVIEWS. GENETICS. ENGLAND OCT 2002, vol. 3, no. 10, October 2002 (2002-10), pages 737-747, ISSN: 1471-0056
- DATABASE EMBL [Online] Secreted polypeptide-related cDNA #33, 17 September 2003 (2003-09-17) retrieved from EBI Database accession no. ACD66744 XP002263161 & US 2003/022279 A1 (MACKAY CHARLES R ET AL.) 30 January 2003 (2003-01-30)

### SEQUENCE LISTING

<110> MERCK-SANTE
   CENTRE NATIONAL DE LA RECHERCHE SCIENTIFIQUE (CNRS)
<120> Insulin-induced gene as therapeutic target in diabetes
<130> BFF 03P0004
<160> 8
<170> PatentIn version 3.1
<210> 1
   <211> 1062
   <212> DNA
   <213> Rattus sp.
<400> 1
   atgttgtgca ccctgttcct gctactgctg gccctgggca tagtgcagac aactcggcca   60
   tgtttccctg gctgccagtg tgaggaagag acgtttggcc tctttgacag tttcagcctg  120
   atccgagtgg actgcagcag cctgggcccc cacattgtgc ctgtgcccat ccctctggac  180
   acagcccacc tggacctgtc ttccaaccgg ctagaaaccg tgaatgagtc agtcctggga  240
   gggccaggct ataccacact ggctggcctg gatctcagtc acaacctcct caccagcatc  300
   acgcccactg ccttctcccg ccttcgctac ctggagtcac tggacctcag tcacaatggc  360
   ctggcagccc tgccagcaga ggttttcacc agctccccct tgagtgatat caacctgagc  420
   cataatcgac ttagagaggt ctcgatatct gccttcacca cccacagcca ggggcgggca  480
   ctgcacgtgg acctatccca caatcttatc caccgcctgc tcccctatcc agccagggcc  540
   agcctgtccg cacctaccat tcagagcctg aacctgtcct ggaaccggct ccgagccgtg  600
   cccgatctcc gagacctacc cctgcgttac ctgagcctgg atgggaaccc tctggctacc  660
   atcaacccag gcgccttcat ggggctggcg ggcctcaccc acctttcact ggcaagccta  720
   cagggtatcc tccagctacc accccatggc ttccgagagc tcccaggcct tcaggtcctg  780
   gacttgtctg gtaaccccaa gctcaagtgg gcaggagccg aggtattttc aggcctgggt  840
   ttgctgcaag aactagacct gtctggctcc agcctggtgc cctgcctgga gacgctgcta  900
   catcacctcc ctgctttaca gagtgtcagt gtaggccaag atgtgcagtg ccggcgtctg  960
   gtacgggagg gtgcctacca ccgccaaccc ggttccagcc ctaaggtagt cctgcactgt 1020
   ggagacaccc aggaatctgc caggggccca gacatcttgt ga                   1062
<210> 2
   <211> 353
   <212> PRT
   <213> Rattus sp.
<400> 2
   Met Leu Cys Thr Leu Phe Leu Leu Leu Leu Ala Leu Gly Ile Val Gln
   1               5                   10                  15
   Thr Thr Arg Pro Cys Phe Pro Gly Cys Gln Cys Glu Glu Glu Thr Phe
               20                  25                  30
   Gly Leu Phe Asp Ser Phe Ser Leu Ile Arg Val Asp Cys Ser Ser Leu
           35                  40                  45
   Gly Pro His Ile Val Pro Val Pro Ile Pro Leu Asp Thr Ala His Leu
       50                  55                  60
   Asp Leu Ser Ser Asn Arg Leu Glu Thr Val Asn Glu Ser Val Leu Gly
   65                  70                  75                  80
   Gly Pro Gly Tyr Thr Thr Leu Ala Gly Leu Asp Leu Ser His Asn Leu
                   85                  90                  95
   Leu Thr Ser Ile Thr Pro Thr Ala Phe Ser Arg Leu Arg Tyr Leu Glu
               100                 105                 110
   Ser Leu Asp Leu Ser His Asn Gly Leu Ala Ala Leu Pro Ala Glu Val
           115                 120                 125
   Phe Thr Ser Ser Pro Leu Ser Asp Ile Asn Leu Ser His Asn Arg Leu
       130                 135                 140
   Arg Glu Val Ser Ile Ser Ala Phe Thr Thr His Ser Gln Gly Arg Ala
   145                 150                 155                 160
   Leu His Val Asp Leu Ser His Asn Leu Ile His Arg Leu Leu Pro Tyr
                   165                 170                 175
   Pro Ala Arg Ala Ser Leu Ser Ala Pro Thr Ile Gln Ser Leu Asn Leu
               180                 185                 190
   Ser Trp Asn Arg Leu Arg Ala Val Pro Asp Leu Arg Asp Leu Pro Leu
       195                 200         205
   Arg Tyr Leu Ser Leu Asp Gly Asn Pro Leu Ala Thr Ile Asn Pro Gly
     210             215             220
   Ala Phe Met Gly Leu Ala Gly Leu Thr His Leu Ser Leu Ala Ser Leu
   225             230             235             240
   Gln Gly Ile Leu Gln Leu Pro Pro His Gly Phe Arg Glu Leu Pro Gly
               245             250             255
   Leu Gln Val Leu Asp Leu Ser Gly Asn Pro Lys Leu Lys Trp Ala Gly
           260             265             270
   Ala Glu Val Phe Ser Gly Leu Gly Leu Leu Gln Glu Leu Asp Leu Ser
       275                 280             285
   Gly Ser Ser Leu Val Pro Leu Pro Glu Thr Leu Leu His His Leu Pro
     290             295             300
   Ala Leu Gln Ser Val Ser Val Gly Gln Asp Val Gln Cys Arg Arg Leu
   305             310             315             320
   Val Arg Glu Gly Ala Val His Arg Gln Pro Gly Ser Ser Pro Lys Val
               325             330             335
   Val Leu His Cys Gly Asp Thr Gln Glu Ser Ala Arg Gly Pro Asp Ile
           340             345             350
   Leu
<210> 3
   <211> 2557
   <212> DNA
   <213> Homo sapiens
<220>
   <221> CDS
   <222> (14)..(1075)
   <223>
<400> 3
   tccagccccc acc atg ccg tgg ccc ctg ctg ctg ctg ctg gcc gtg agt    49
   Met Pro Trp Pro Leu Leu Leu Leu Leu Ala Val Ser
   1           5                   10
   ggg gcc cag aca acc cgg cca tgc ttc ccc ggg tgc caa tgc gag gtg      97
   Gly Ala Gln Thr Thr Arg Pro Cys Phe Pro Gly Cys Gln Cys Glu Val
       15                  20                  25
   gag acc ttc ggc ctt ttc gac agc ttc agc ctg act cgg gtg gat tgt     145
   Glu Thr Phe Gly Leu Phe Asp Ser Phe Ser Leu Thr Arg Val Asp Cys
     30                  35                  40
   agc ggc ctg ggc ccc cac atc atg ccg gtg ccc atc cct ctg gac aca     193
   Ser Gly Leu Gly Pro His Ile Met Pro Val Pro Ile Pro Leu Asp Thr
   45                  50                  55                  60
   gcc cac ttg gac ctg tcc tcc aac cgg ctg gag atg gtg aat gag tcg     241
   Ala His Leu Asp Leu Ser Ser Asn Arg Leu Glu Met Val Asn Glu Ser
               65                  70                  75
   gtg ttg gcg ggg ccg ggc tac acg acg ttg gct ggc ctg gat ctc agc     289
   Val Leu Ala Gly Pro Gly Tyr Thr Thr Leu Ala Gly Leu Asp Leu Ser
           80                  85                  90
   cac aac ctg ctc acc agc atc tca ccc act gcc ttc tcc cgc ctt cgc     337
   His Asn Leu Leu Thr Ser Ile Ser Pro Thr Ala Phe Ser Arg Leu Arg
       95                  100                 105
   tac ctg gag tcg ctt gac ctc agc cac aat ggc ctg rca gcc ctg cca     385
   Tyr Leu Glu Ser Leu Asp Leu Ser His Asn Gly Leu Xaa Ala Leu Pro
     110                 115                 120
   gcc gag agc ttc acc agc tca ccc ctg agc gac gtg aac ctt agc cac     433
   Ala Glu Ser Phe Thr Ser Ser Pro Leu Ser Asp Val Asn Leu Ser His
   125                 130                 135                 140
   aac cag ctc cgg gag gtc tca gtg tct gcc ttc acg acg cac agt cag     481
   Asn Gln Leu Arg Glu Val Ser Val Ser Ala Phe Thr Thr His Ser Gln
               145                 150                 155
   ggc cgg gca cta cac gtg gac ctc tcc cac aac ctc att cac cgc ctc     529
   Gly Arg Ala Leu His Val Asp Leu Ser His Asn Leu Ile His Arg Leu
           160                 165                 170
   gtg ccc cac ccc acg agg gcc ggc ctg cct gcg ccc acc att cag agc     577
   Val Pro His Pro Thr Arg Ala Gly Leu Pro Ala Pro Thr Ile Gln Ser
       175                 180                 185
   ctg aac ctg gcc tgg aac cgg ctc cat gcc gtg ccc aac ctc cga gac     625
   Leu Asn Leu Ala Trp Asn Arg Leu His Ala Val Pro Asn Leu Arg Asp
     190                 195                 200
   ttg ccc ctg cgc tac ctg agc ctg gat ggg aac cct cta gct gtc att     673
   Leu Pro Leu Arg Tyr Leu Ser Leu Asp Gly Asn Pro Leu Ala Val Ile
   205                 210                 215                 220
   ggt ccg ggt gcc ttc gcg ggg ctg gga ggc ctt aca cac ctg tct ctg     721
   Gly Pro Gly Ala Phe Ala Gly Leu Gly Gly Leu Thr His Leu Ser Leu
               225                 230                 235
   gcc agc ctg cag agg ctc cct gag ctg gcg ccc agt ggc ttc cgt gag     769
   Ala Ser Leu Gln Arg Leu Pro Glu Leu Ala Pro Ser Gly Phe Arg Glu
           240                 245                 250
   cta ccg ggc ctg cag gtc ctg gac ctg tcg ggc aac ccc aag ctt aac    817
   Leu Pro Gly Leu Gln Val Leu Asp Leu Ser Gly Asn Pro Lys Leu Asn
       255         260         265
   tgg gca gga gct gag gtg ttt tca ggc ctg agc tcc ctg cag gag ctg    865
   Trp Ala Gly Ala Glu Val Phe Ser Gly Leu Ser Ser Leu Gln Glu Leu
     270         275         280
   gac ctt tcg ggc acc aac ctg gtg ccc ctg cct gag gcg ctg ctc ctc    913
   Asp Leu Ser Gly Thr Asn Leu Val Pro Leu Pro Glu Ala Leu Leu Leu
   285         290         295         300
   cac ctc ccg gca ctg cag agc gtc agc gtg ggc cag gat gtg cgg tgc    961
   His Leu Pro Ala Leu Gln Ser Val Ser Val Gly Gln Asp Val Arg Cys
           305         310         315
   cgg cgc ctg gtg cgg gag ggc acc tac ccc cgg agg cct ggc tcc agc    1009
   Arg Arg Leu Val Arg Glu Gly Thr Tyr Pro Arg Arg Pro Gly Ser Ser
       320         325         330
   ccc aag gtg gcc ctg cac tgc gta gac acc cgg gaa tct gct gcc agg    1057
   Pro Lys Val Ala Leu His Cys Val Asp Thr Arg Glu Ser Ala Ala Arg
       335         340         345
   ggc ccc acc atc ttg tga caaatggtgt ggcccagggc cacataacag    1105
   Gly Pro Thr Ile Leu
     350
   actgctgtcc tgggctgcct caggtcccga gtaacttatg ttcaatgtgc caacaccagt  1165
   ggggagcccg caggcctatg tggcagcgtc accacaggag ttgtgggcct aggagaggct  1225
   ttggacctgg gagccacacc taggagcaaa gtctcacccc tttgtctacg ttgcttcccc  1285
   aaaccatgag cagagggact tcgatgccaa accagactcg ggtcccctcc tgcttccctt  1345
   ccccacttat cccccaagtg ccttccctca tgcctgggcc ggctgacccg caatgggcag  1405
   agggtgggtg gacccctgct gcagggcaga gttcaggtcc actgggctga gtgtcccttg  1465
   ggcccatggc ccagtcactc aggggcgagt ttcttttcta acatagccct ttctttgcca  1525
   tgaggccatg aggcccgctt catccttttc tatttcccta gaaccttaat ggtagaagga  1585
   attgcaaaga atcaagtcca cccttctcat gtgacagatg gggaaactga ggccttgaga  1645
   aggaaaaagg ctaatctaag ttcctgcggg cagtggcatg actggagcac agcctcctgc  1705
   ctcccagccc ggacccaatg cactttcttg tctcctctaa taagccccac cctccccgcc  1765
   tgggctcccc ttgctgccct tgcctgttcc ccattagcac aggagtagca gcagcaggac  1825
   aggcaagagc ctcacaagtg ggactctggg cctctgacca gctgtgcggc atgggctaag  1885
   tcactctgcc cttcggagcc tctggaagct tagggcacat tggttccagc ctagccagtt  1945
   tctcaccctg ggttggggtc ccccagcatc cagactggaa acctacccat tttcccctga  2005
   gcatcctcta gatgctgccc caaggagttg ctgcagttct ggagcctcat ctggctggga  2065
   tctccaaggg gcctcctgga ttcagtcccc actggccctg agcacgacag cccttcttac  2125
   cctcccagga atgccgtgaa aggagacaag gtctgcccga cccatgtcta tgctctaccc  2185
   cagggtagca tctcagcttc cgaaccctgg gctgtttcct tagtcttcat tttataaaag  2245
   ttgttgcctt tttaacggag tgtcactttc aaccggcctc ccctacccct gctggccggg  2305
   gatggagaca tgtcatttgt aaaagcagaa aaaggttgca tttgttcact tttgtaatat  2365
   tgtcctgggc ctgtgttggg gtgttggggg aagctgggca tcagtggcca catgggcatc  2425
   aggggctggc cccacagaga ccccacaggg cagtgagctc tgtcttcccc cacctgccta  2485
   gcccatcatc tatctaaccg gtccttgatt taataaacac tataaaaagt taaaaaaaaa  2545
   aaaaaaaaaa aa                                2557
<210> 4
   <211> 353
   <212> PRT
   <213> Homo sapiens
<220>
   <221> misc_feature
   <222> (121)..(121)
   <223> 'Xaa' in position 121 represents Ala or Thr.
<400> 4
   Met Pro Trp Pro Leu Leu Leu Leu Leu Ala Val Ser Gly Ala Gln Thr
   1               5                   10                  15
   Thr Arg Pro Cys Phe Pro Gly Cys Gln Cys Glu Val Glu Thr Phe Gly
               20                  25                  30
   Leu Phe Asp Ser Phe Ser Leu Thr Arg Val Asp Cys Ser Gly Leu Gly
           35                  40                  45
   Pro His Ile Met Pro Val Pro Ile Pro Leu Asp Thr Ala His Leu Asp
       50                  55                  60
   Leu Ser Ser Asn Arg Leu Glu Met Val Asn Glu Ser Val Leu Ala Gly
   65                  70                  75                  80
   Pro Gly Tyr Thr Thr Leu Ala Gly Leu Asp Leu Ser His Asn Leu Leu
                   85                  90                  95
   Thr Ser Ile Ser Pro Thr Ala Phe Ser Arg Leu Arg Tyr Leu Glu Ser
               100                 105                 110
   Leu Asp Leu Ser His Asn Gly Leu Xaa Ala Leu Pro Ala Glu Ser Phe
           115                 120                 125
   Thr Ser Ser Pro Leu Ser Asp Val Asn Leu Ser His Asn Gln Leu Arg
   130 135 140
   Glu Val Ser Val Ser Ala Phe Thr Thr His Ser Gln Gly Arg Ala Leu
   145 150 155 160
   His Val Asp Leu Ser His Asn Leu Ile His Arg Leu Val Pro His Pro
   165 170 175
   Thr Arg Ala Gly Leu Pro Ala Pro Thr Ile Gln Ser Leu Asn Leu Ala
   180 185 190
   Trp Asn Arg Leu His Ala Val Pro Asn Leu Arg Asp Leu Pro Leu Arg
   195 200 205
   Tyr Leu Ser Leu Asp Gly Asn Pro Leu Ala Val Ile Gly Pro Gly Ala
   210 215 220
   Phe Ala Gly Leu Gly Gly Leu Thr His Leu Ser Leu Ala Ser Leu Gln
   225 230 235 240
   Arg Leu Pro Glu Leu Ala Pro Ser Gly Phe Arg Glu Leu Pro Gly Leu
   245 250 255
   Gln Val Leu Asp Leu Ser Gly Asn Pro Lys Leu Asn Trp Ala Gly Ala
   260 265 270
   Glu Val Phe Ser Gly Leu Ser Ser Leu Gln Glu Leu Asp Leu Ser Gly
   275 280 285
   Thr Asn Leu Val Pro Leu Pro Glu Ala Leu Leu Leu His Leu Pro Ala
   290 295 300
   Leu Gln Ser Val Ser Val Gly Gln Asp Val Arg Cys Arg Arg Leu Val
   305 310 315 320
   Arg Glu Gly Thr Tyr Pro Arg Arg Pro Gly Ser Ser Pro Lys Val Ala
   325 330 335
   Leu His Cys Val Asp Thr Arg Glu Ser Ala Ala Arg Gly Pro Thr Ile
   340 345 350
   Leu
<210> 5
   <211> 25
   <212> DNA
   <213> Artificial : primer
<400> 5
   attaaccctc actaaatgct gggtg 25
<210> 6
   <211> 31
   <212> DNA
   <213> Artificial : primer
<400> 6
   cattatgctg agtgatatct tttttttttt g 31
<210> 7
   <211> 22
   <212> DNA
   <213> Artificial : primer
<400> 7
   acgcgggggg gtcgcctagg tg 22
<210> 8
   <211> 29
   <212> DNA
   <213> Artificial : primer
<400> 8

## Description

The invention relates to the identification of the E2IG4 gene as a therapeutic target for the treatment of diabetes or its complications, obesity or insulin resistance.

Diabetes is a heterogeneous metabolic disease which is currently increasing rapidly throughout the world, representing a serious public health problem. Diabetes is characterised by an increase in blood glucose coupled with a defective secretion of insulin by the pancreatic β cells or with a defective uptake of glucose by the peripheral tissues such as the liver, muscle or adipose tissue, which defines insulin resistance (Mauvais-Jarvis F., Kahn C.R., Diabetes Metab., 2000, 26(6), 433-48). In addition, a large proportion of obese subjects develop insulin resistance and possibly diabetes.

The inventors were interested in searching for novel therapeutic targets exploitable for the treatment of these pathological conditions.

Using the differential display technique, they compared the expression profiles of all the hepatic genes in the rat, in the absence or presence of insulin, and thereby succeeded in identifying a gene whose expression is induced rapidly by insulin.

The inventors first called this gene the EIIH gene (Early Insulin Induced Hepatic gene). This gene in fact proves to be the homologue in the rat of the gene described in man as the E2IG4 gene (E-2 induced gene) (Charpentier et al., Cancer Res., 2000, 60(21), 5977-83). The cDNA sequence cloned in the rat by the inventors is shown in the attached sequence listing (SEQ ID No.1). The corresponding protein sequence (of 353 amino acids) is shown in SEQ ID No. 2. The cDNA sequence cloned in man by Charpentier et al., 2000, is shown in SEQ ID No. 3 and the corresponding protein sequence in SEQ ID No. 4.

In the context of the present description, E2IG4 gene is taken to mean not only the human sequence but also the homologues in other species, for example the rat or the mouse or any other mammal. The designations EIIH gene and E21G4 gene will moreover be used interchangeably. Likewise, the protein or expression product of this gene will arbitrarily be referred to as EIIH protein or E2IG4 protein.

The aim of the present invention is to use this gene or the expression product of this gene as a therapeutic target for the treatment of diabetes or its complications, obesity or insulin resistance. Among the complications in question, there may be mentioned macrovascular complications such as atherosclerosis, or microvascular complications such as retinopathy, nephropathy and neuropathy.

In fact, the experiments carried out show that the accumulation kinetics of the mRNA of this gene are early and precede those of glucokinase, strongly suggesting that the expression of this gene plays a role in insulin signalling mechanisms and in glucose uptake and metabolism. Identification of the E2IG4 protein as an insulin responder leads to a number of therapeutic and diagnostic applications that are subjects of the invention.

The invention covers the use of the E2IG4 protein or a nucleic acid coding for this protein (within the framework of a gene therapy) for the manufacture of a drug for the treatment of diabetes or its complications, obesity or insulin resistance.

The pharmaceutical compositions comprising an E2IG4 protein or a nucleic acid coding for this protein, in association with a pharmaceutically acceptable vehicle, also form part of the invention.

Described is an *in vitro* method of screening or identifying compounds useful in the treatment of diabetes or its complications, obesity or insulin resistance, in which at least one test compound is brought into contact with a cell capable of expressing the E21G4 gene, and the level of expression of this gene is evaluated, an increase in the level of expression of this gene being indicative of a compound useful in the treatment of diabetes or its complications, obesity or insulin resistance.

Further described is *in vitro* method of screening or identifying compounds useful in the treatment of diabetes or its complications, obesity or insulin resistance, in which at least one test compound is brought into contact with a cell capable of expressing a reporter gene associated as an operator with the promoter of the E21G4 gene, and the level of expression of the reporter gene is evaluated, an increase in the level of expression of this gene being indicative of a compound useful in the treatment of diabetes or its complications, obesity or insulin resistance.

This method can be implemented by a variety of techniques well known to those skilled in the art, for example by using a cell transfected with a nucleic acid comprising the sequence SEQ ID No. 1.

Further described is an *in vitro* method for the diagnosis or prognosis of diabetes, obesity or insulin resistance in a subject, in which the level of expression of the product of the E2IG4 gene is determined in a subject's biological sample, a modification of the level of expression compared with a control subject's biological sample being indicative of the development or an increased risk of development of diabetes, obesity or insulin resistance in the said subject.

The level of expression of the product of the E2IG4 gene can be determined in different ways, preferably by evaluating the amount of E21G4 protein in a subject's biological sample, for example by the conventional immunoassay techniques. It is also possible to measure the level of expression of mRNA transcribed from this gene.

The aim is to detect a decrease in the level of expression of the product of the E21G4 gene compared with the control subject, a decrease being indicative of the development or an increased risk of development of diabetes, obesity or insulin resistance.

### Gene therapy

Gene therapy can be carried out,in which a nucleic acid coding for the E2IG4 protein is administered to a patient under conditions such that the protein is expressed *in vivo* by those of the patient's cells into which the nucleic acid has been transferred.

According to the invention the nucleic acid administered is the nucleotide sequence SEQ ID No. 3 or No. 1. Described but not part of the invention is the use of any homologous or similar sequence defined as follows:
i) sequences at least 70% similar to one of the identified sequences;
ii) sequences hybridising with one of the said identified sequences or its complementary sequence under stringent hybridisation conditions; or
iii) sequences coding for a polypeptide as defined below.

A homologous nucleotide sequence according to the invention is preferably at least 75%, particularly preferably at least 85% or at least 90% and very particularly preferably at least 95% similar to the identified sequences.

Preferably, such a homologous nucleotide sequence hybridises specifically with the complementary sequences of one of the identified sequences under stringent conditions. The parameters defining the stringency conditions depend on the temperature at which 50% of the matching strands separate (Tm).

For sequences comprising more than 30 bases, Tm is defined by the following relationship: Tm = 81.5 + 0.41 (%G+C) + 16.6log(cation concentration) - 0.63(%formamide) - (600/number of bases) (Sambrook et al., 1989, Molecular Cloning: A Laboratory Manual, 2nd edition, Cold Spring Harbor Laboratory Press, Cold Spring Harbor, New York).

For sequences with a length of less than 30 bases, Tm is defined by the following relationship: Tm = 4(G+C) + 2(A+T).

Under appropriate stringency conditions in which the non-specific sequences do not hybridise, the hybridisation temperature can preferably be 5 to 10°C below Tm and the hybridisation buffers used are preferably solutions of high ionic strength, for example a 6 x SSC solution.

The term "similar sequences" employed above refers to the perfect resemblance or identity between the compared nucleotides, but also to the imperfect resemblance classed as similarity. This search for similarities in nucleic acid sequences distinguishes for example purines from pyrimidines.

A homologous nucleotide sequence therefore includes any nucleotide sequence which differs from one of the identified sequences by the mutation, insertion, deletion or substitution of one or more bases or by the degeneracy of the genetic code.

Such homologous sequences include gene sequences of mammals other than man, preferably those of primates, cattle, sheep or pigs, or those of other rodents, as well as the allelic variants.

The sequence coding for the E2IG4 protein is preferably associated with components for regulating its expression, such as a promoter sequence.

Such a nucleic acid can be especially in the form of a DNA vector, for example a plasmid vector.

The DNA vector can be introduced *in vivo* by any technique known to those skilled in the art. In particular, the DNA vector can be introduced *in vivo* in a naked form, i.e. without the aid of any vehicle or system to facilitate the transfection of the vector into the cells (EP 465 529).

It is also possible to employ a gene gun, for example by depositing the DNA on the surface of "gold" particles and shooting them so that the DNA penetrates the patient's skin. Injection with a liquid gel can also be employed to transfect both skin, muscle, fatty tissue and mammary tissue.

Microinjection, electroporation and calcium phosphate precipitation techniques, and formulations using nanocapsules or liposomes, are other available techniques.

Biodegradable polyalkyl cyanoacrylate nanoparticles are particularly advantageous. In the case of liposomes, the use of cationic lipids favours the encapsulation of negatively charged nucleic acids and facilitates fusion with negatively charged cell membranes.

Alternatively, the vector can be in the form of a recombinant virus comprising, inserted in its genome, a nucleic acid sequence coding for the said peptide(s).

The viral vector can preferably be selected from an adenovirus, a retrovirus, particularly a lentivirus, and an adeno-associated virus (AAV), a herpes virus, a cytomegalovirus (CMV), a vaccine virus, etc.

Lentivirus vectors have been described for example by Firat et al., (2002), J. Gen. Ther., 4, 38-45.

Advantageously, the recombinant virus is a defective virus. The term "defective virus" denotes a virus incapable of replicating in a target cell. In general, the genome of defective viruses is devoid of at least the sequences necessary for replication of the said virus in the infected cell. These regions can be either removed or rendered non-functional or substituted by other sequences, particularly by the nucleic acid coding for the peptide of interest. Nevertheless, the defective virus preferably retains the sequences of its genome which are necessary for encapsulation of the viral particles.

### Protein or peptide therapy

In another embodiment, E2IG4 protein can be increased in the patient by administering an exogenous E2IG4 protein which has preferably been purified and has optionally been chemically or enzymatically modified to improve its stability or bioavailability.

According to the invention "*exogenous E2IG4 protein"* is taken to mean the protein of amino acid sequence SEQ ID No. 4 or No. 2. Also described but not part of the invention is the use of any variant, homologous or derived sequences defined as sequences that are at least 70%, preferably at least 80%, particularly preferably at least 90% or at least 95% similar to the reference sequence.

These sequences can also be defined as comprising the sequences encoded by a nucleic acid sequence hybridising with the reference sequence or its complementary sequence under stringent hybridisation conditions.

The term "similar" refers to the perfect resemblance or identity between the compared amino acids, but also to the imperfect resemblance classed as similarity. This search for similarities in a polypeptide sequence takes conservative substitutions into account; these are substitutions of amino acids of the same class, such as substitutions of amino acids with uncharged side chains (for example asparagine, glutamine, serine, threonine and tyrosine), amino acids with basic side chains (for example lysine, arginine and histidine), amino acids with acidic side chains (for example aspartic acid and glutamic acid) or amino acids with non-polar side chains (for example glycine, alanine, valine, leucine, isoleucine, proline, phenylalanine, methionine, tryptophan and cysteine). More generally, *"variant, homologous or derived amino acid sequence"* is therefore taken to mean any amino acid sequence which differs from the reference sequence by the substitution, deletion and/or insertion of one or more amino acids, preferably a reduced number of amino acids, especially by the substitution of natural amino acids by unnatural amino acids or pseudo-amino acids in positions such that these modifications do not have a significant effect on the biological activity of the protein.

The homology is generally determined by using a sequence analysis software (for example Sequence Analysis Software Package of the Genetics Computer Group, University of Wisconsin Biotechnology Center, 1710 University Avenue, Madison, WI 53705). Similar amino acid sequences are aligned so as to maximise the degree of homology (i.e. identity or similarity, as defined above). To this end it may be necessary artificially to introduce gaps into the sequence. When the alignment has been optimised, the degree of homology is established by recording all the positions for which the amino acids of the two compared sequences are identical, relative to the total number of positions.

Preferably, the "variant", "homologous" or "derived" peptides have the same length as the reference sequences.

The E21G4 protein can be synthesised by any of the methods well known to those skilled in the art, for example by the synthetic chemistry techniques such as Merrifield synthesis, which is advantageous for reasons of purity, antigenic specificity and absence of unwanted by-products and for its ease of production.

A recombinant protein can also be produced by a process in which a vector containing a nucleic acid comprising one of the identified sequences, or a homologous sequence, is transferred into a host cell, which is cultured under conditions that allow the expression of the corresponding polypeptide.

The protein produced can then be recovered and purified.

The purification processes used are known to those skilled in the art. The recombinant polypeptide obtained can be purified from cell lysates and extracts, from the culture medium supernatant, by methods used individually or in combination, such as fractionation, chromatography methods, immunoaffinity techniques with the aid of specific monoclonal or polyclonal antibodies, etc.

### Modulators and screening methods

Modulation of the activity of the E21G4 protein, namely its activation or inhibition, can be effected using a variety of modulators identified for example by screening methods.

Described is thus a method of screening or identifying compounds useful in the treatment of diabetes or its complications, obesity or insulin resistance, in which at least one test compound is brought into contact with a cell capable of expressing the E2IG4 gene, and the level of expression of this gene is evaluated, a modulation (namely an increase or decrease, preferably an increase) of the level of expression of this gene being indicative of a compound useful in the treatment of diabetes or its complications, obesity or insulin resistance.

The level of expression of the E2IG4 gene in the cells exposed to the test compound can be compared with the control level of expression of the cells not exposed to the compound.

The test compound can be of any type. It can consist of natural or synthetic compounds or mixtures thereof. It can also be a substance of defined structure or unknown structure, for example a biological extract.

The cells used in the screening methods can be cells that naturally express E2IG4, such as FAO, H4IIE, AtT20, MCF-7 or INIS-1 cells, or they can be host cells transfected in a transitory or stable manner with expression vectors of the product of the E21G4 gene. These cells can be obtained by the introduction, into prokaryotic or eukaryotic host cells, of a nucleotide sequence inserted in a vector as defined above, followed by the culture of the said cells under conditions that allow the replication and/or expression of the transfected nucleotide sequence.

Examples of host cells include especially mammalian cells, such as CHO, COS-7, 293 or MDCK cells, insect cells, such as SF9 cells, bacteria, such as E. coli, and yeast strains.

The level of expression can be evaluated by determining the level of transcription of the gene or the level of translation of the protein encoded by this E2IG4 gene, either directly or, for example, via a reporter gene.

The most common tests for following the transcription (i.e. determining the level of transcription) of the target gene (in this case the E21G4 gene) or the reporter gene are based on the northern blotting technique. The tests for following the translation (i.e. determining the level of translation) of the E2IG4 protein or the reporter protein can be based especially on the immunoassay techniques or can use the fluorimetric, luminescent or other techniques for detecting reporter proteins (green fluorescent protein, GFP; luciferase; chloramphenicol acetyltransferase, CAT; etc.).

The immunoassay techniques can be carried out according to various formats well known to those skilled in the art, for example by ELISA, radioimmunoassay, *in situ* immunoassays, western blotting, immunofluorescence, etc. The anti-E2IG4 protein antibodies useful for detecting the E2IG4 protein can be produced as described below.

### Antibodies

Further described is the production of anti-E2IG4 protein antibodies, which can be useful in the above-described screening methods or as modulators of interest, particularly as inhibitors of the activity of the E2IG4 protein, all the more so because they possess a blocking capacity. They are therefore referred to as inhibitory antibodies.

The antibodies can be monoclonal antibodies or polyclonal, preferably monospecific sera.

The monoclonal antibodies can be obtained by the conventional method of lymphocyte fusion and hybridoma culture described by Köhler and Milstein, Nature, 1975, 256, 495-497. Other methods of preparing monoclonal antibodies are also known (Harlow et al., Eds., 1988 "Antibodies: A Laboratory Manual"). The monoclonal antibodies can be prepared by immunising a mammal (for example a mouse, a rat, a rabbit or even a human, etc.) and using the lymphocyte fusion technique to produce hybridomas (Köhler and Milstein, 1975).

Alternative techniques to this customary technique exist. For example, it is possible to produce monoclonal antibodies by the expression of a nucleic acid cloned from a hybridoma. It is also possible to produce antibodies by the phage display technique, antibody cDNAs being introduced into vectors which are typically filamentous phages having V-gene libraries on the surface of the phage (for example fUSES for *E*. *coli,* Scott, J.K. and Smith, G.P., Science, 1990, 249, 386-390). Protocols for the construction of these antibody libraries are described in Marks et al., 1991, J. Mol. Biol., 222, 581-597.

The polyclonal antibodies can be obtained from the serum of an animal immunised against an antigen of a peptide nature by the customary procedures. The antigen used can thus be the E21G4 protein or an appropriate peptide fragment thereof that is capable of being coupled via a reactive residue with a protein or another peptide. Rabbits are immunised with the equivalent of 1 mg of the peptide antigen. At four-week intervals the animals are treated with 200 µg injections. of antigen and bled 10 to 14 days later. After the third injection, the antiserum is examined in order to determine its capacity to bind to the iodine-radiolabelled- peptide antigen, prepared by the chloramine-T method, and is then purified by ion exchange chromatography on a carboxymethyl cellulose (CMC) column. The antibody molecules are then collected from the mammals and isolated to the desired concentration by the methods well known to those skilled in the art, for example by using DEAE Sephadex to give the IgG fraction.

### Other inhibitors

The modulators also include peptide fragments of the E2IG4 protein which inhibit the activity of the E2IG4 protein by interaction therewith or with one of its effectors.

### Repression of the transcription

The expression of E2IG4 is modulated by inhibiting or repressing the transcription of the gene. Those skilled in the art know how to choose the most appropriate strategy for this purpose.

For example, antisense nucleic acids, including ribozymes, can be used. An antisense therapy generally uses a vector, such as a viral vector, which carries the antisense sequence, the inhibition then being generally stable since the vector will integrate into the genome. It is also possible to use antisense oligonucleotides to procure a transitory inhibition of the expression.

A further possibility is to utilise the technology of interfering RNAs (siRNAs), which prevents a gene from producing a functional protein by destroying the messenger RNA (Bass, Cell, 2000, 101, 235-238; Sharp, Genes Dev., 2001, 15, 485-490).

### Pharmaceutical compositions

Described is a pharmaceutical composition which comprises, as active principle, a modulator of the expression or activity of the E2IG4 gene, together with one or more pharmaceutically acceptable excipients. As described above, this modulator can be a chemically synthesised compound, an antisense or interfering RNA or an anti-E2IG4 antibody.

Subject of the invention is a pharmaceutical composition which comprises, as active principle, an E2IG4 protein or a nucleic acid comprising a sequence coding for this protein namely SEQ ID NO: 1 and 3, together with one or more pharmaceutically acceptable excipients.

"Pharmaceutically acceptable excipient" or "vehicle" is taken to mean any solvent, dispersion medium, absorption retarders, etc. which do not produce a secondary reaction, for example an allergic reaction, in the human or animal.

The dosage naturally depends on the active principle in question, the mode of administration, the therapeutic indication and the patient's age and condition.

The dose of protein or antibody is preferably 0.1 to 250 mg/kg per day and particularly preferably 1 to 100 mg/kg per day.

When the pharmaceutical compositions comprise nucleic acids, the doses of nucleic acid (sequence or vector) to be administered are also adapted in particular according to the mode of administration, the target pathological condition and the treatment time. In general, when recombinant viruses are used, these are formulated and administered as doses of from about 10⁴ to 10¹⁴ pfu/ml and preferably from 10⁶ to 10¹⁰ pfu/ml. The term "pfu" (plaque forming unit) corresponds to the infectivity of a viral solution and can be determined by infecting an appropriate cell culture and measuring the number of infected cell plaques, generally after 48 hours. The techniques for determining the pfu titre of a viral solution are well described in the literature.

Where parenteral administration is envisaged, more particularly by injection, the compositions of the invention comprising the active principle(s) take the form of injectable solutions and suspensions packaged in vials or bottles for slow perfusion. In particular, the injection can be given subcutaneously, intramuscularly or intravenously.

In the case of oral administration, the compositions of the invention take the form of gelatin capsules, effervescent tablets, uncoated or coated tablets, sachets, dragees, vials or solutions to be taken orally, microgranules or prolonged release forms.

The forms for parenteral administration are obtained in conventional manner by mixing the active principle(s) with buffers, stabilisers, preservatives, solubilisers, isotonic agents and suspending agents. In accordance with the known techniques, these mixtures are subsequently sterilised and then packed in the form of intravenous injections.

Buffers that may be used by those skilled in the art are buffers based on organic phosphate salts.

Examples of suspending agents include methyl cellulose, hydroxyethyl cellulose, hydroxypropyl cellulose, acacia and sodium carboxymethyl cellulose.

Moreover, stabilisers useful according to the invention are sodium sulfite and sodium metasulfite, while sodium p-hydroxybenzoate, sorbic acid, cresol and chlorocresol may be mentioned as preservatives. For the preparation of an oral solution or suspension, the active principles are dissolved or suspended in an appropriate vehicle with a dispersant, a humectant, a suspending agent (for example polyvinylpyrrolidone), a preservative (such as methylparaben or propylparaben), a taste corrector or a colourant.

For the preparation of microcapsules, the active principles are combined with appropriate diluents, appropriate stabilisers, agents for promoting the prolonged release of the active substances, or any other type of additive for forming a central core, which is then coated with an appropriate polymer (for example a water-soluble or water-insoluble resin). The techniques known to those skilled in the art will be used for this purpose.

The microcapsules obtained are then optionally formulated in appropriate dosage units.

Ocular administration can also be envisaged.

In this case the pharmaceutical composition of the invention takes the form of an ophthalmic composition for local administration to the eye, for example an eye lotion or eye cream.

The modulators or protein compounds can also be formulated as liposomes. The liposomes are formed from phospholipids, which are dispersed in an aqueous medium and spontaneously form multilamellar concentric bilayer vesicles. These vesicles generally have a diameter of from 25 nm to 4 µm and can be sonicated, resulting in the formation of smaller monolamellar vesicles, with a diameter of from 200 to 500 A, containing an aqueous solution in their core.

The liposomes can be particularly advantageous for administering the drug to a precise target cell or tissue. This can be done by coupling the lipids chemically with target molecules, such as target peptides (for example hormones), or antibodies.

### Diagnostic applications

The identification of the E2IG4 gene as an insulin responder gene opens up applications for the diagnosis or prognosis of diabetes, obesity or insulin resistance in a subject.

In general, the term "diagnosis" is taken to mean the determination or confirmation of a pathological condition in a subject.

The subject or patient can be a mammal, more particularly a human, irrespective of age, sex and state of health. The test subject can be asymptomatic or can be considered susceptible or predisposed to the development of diabetes, obesity or insulin resistance, especially on the grounds of family history. In such cases the term "prognosis" will be used preferentially.

The "control" subject can be a healthy subject. In the case where it is desired to follow the development of one of the pathological conditions mentioned, it is useful to determine, at a given time, the level of expression of the product of the E2IG4 gene in a test subject, and then to redetermine this level of expression at another, later time (it being possible for the time interval to be in the order of several weeks, months or even years). The "control" subject in this second determination is then the "test" subject in the first determination.

The level of expression of the product of the E2IG4 gene can be determined in different ways, preferably by detecting and/or quantifying the E2IG4 protein in a subject's biological sample.

"Biological sample" is understood in particular as meaning blood, serum, urine or a tissue biopsy.

The amount of E2IG4 protein can be determined for example by the conventional immunoassay techniques, including competitive binding assays, direct reaction assays or sandwich-type assays. Western blotting, ELISA, RIA, immunoprecipitation, etc. may be mentioned in particular. The reactions generally involve disclosing markers such as fluorescent, radioactive or enzymatic markers. These immunoassay techniques use an antibody directed against the E2IG4 protein, as described above, preferably a monoclonal antibody.

It is also possible to measure the level of expression of mRNA transcribed from the E21G4 gene. This is generally done by extracting the mRNA from a biological sample (for example a cell or tissue sample) according to the standard techniques, and then subjecting this mRNA to hybridisation techniques (for example of the northern blotting type) and/or amplification techniques (for example PCR) with the aid of oligonucleotides that hybridise specifically with the sequence of the E21G4 gene. The hybridisation complexes formed, or the amplification products, are then detected with the aid of fluorescent, radioactive, enzymatic or other ligands.

The Examples and Figures which follow illustrate the invention without limiting its scope.

### LEGEND TO THE FIGURES:

**Figure 1**: Figure 1 is a graph showing the measurement of the effect of insulin on the level of expression of a control gene (hepatic glucokinase) in STZ rats.
**Figure 2**: Figure 2 is a graph showing the measurement of the effect of insulin on the level of expression of the EIIH gene in STZ rats.

### EXAMPLES:

### Example 1:

### Identification of the insulin-induced EIIH gene by differential display, and cloning of this gene

The inventors chose models developed from 12-day-old suckled rats (12DR) which had never been exposed to effective levels of insulin.

Several observations relating to the effect of insulin on the expression of the glucokinase gene governed this choice of experimental models. Glucokinase is used in this study as a reference and control for the action of insulin on the liver.

It has been reported in particular that, in the liver, insulin controls the expression of glucokinase at the transcriptional level (Magnuson et al., 1989, Proc. Natl. Acad. Sci. USA, 86(13), 4838-42).

Furthermore, in the rat, hepatic glucokinase appears for the first time during the suckling/weaning transition (animal aged between 2 and 3 weeks) (Lynedjian et al., 1987, J. Biol. Chem., 262(13), 6032-8). The necessary protein factors are therefore expressed in this period.

In the culture of rat hepatocytes which have never expressed glucokinase, the addition of insulin induces the accumulation of the specific messenger RNAs with a latency period of from 18 to 24 hours. It has been shown by Bossard et al., 1994, Eur. J. Biochem., 223(2), 371-80, that this delay is necessary for the translation of one or more insulin-regulated proteins.

On the basis of these observations, the inventors were interested in the action of insulin on the expression of target genes, such as glucokinase, via effectors which have to be neosynthesised at the time of the first induction of the gene. To identify these effectors, the inventors chose to study all the genes induced in this particular period in two complementary models:
- *in vivo* model: The administration of an oral dose of glucose to 12-day-old suckled rats (12DR) leads to an increase in their insulinaemia, causing the induction of all the insulin-dependent genes. By removing their livers at different times, it is possible to follow the accumulation of the messenger RNAs of the insulin-stimulated genes.
- *in vitro* model: The hepatocytes of 12-day-old rats are cultured under basal conditions without insulin and without glucose. The addition of insulin to the culture medium induces the gradual accumulation of the messenger RNAs of the genes regulated by this hormone.

The complementarity of these two models has several advantages:
- The use of an *in vivo* model enables the genes to be studied in the animal under physiological conditions.
- The *in vitro* model enables the insulin-induced genes to be observed independently of the effect of glucose.

Thus, by choosing the genes induced both *in vitro* and *in vivo,* the inventors selected the genes induced by insulin alone.

### Materials and methods

### a) Primary culture of the hepatocytes and preparation of the RNAs:

6/8 ten-day-old animals (Wistar rats) are anaesthetised with pento-barbital, the livers are perfused and the hepatocytes are isolated as described in Narkewiez et al., 1990, Biochem. J., 271(3), 585-9. The cells are inoculated at a density of 8.10⁶ cells per 100 mm culture dish into M199 medium (Gibco) comprising 0.1% of BSA, antibiotics and 2% of Ultroser. After four hours of adhesion, the medium is replaced with glucose-free M199 culture medium supplemented with antibiotics, lactate and pyruvate, and insulin (100 nM). Culture is stopped at different times (between 0 and 24 hours) after the addition of insulin. The cells are washed twice in PBS and then lysed in a buffer for the preparation of RNAs according to the protocol described by Chirgwin et al., 1979, Biochemistry, 18(24), 5294-9.

### b) Removal of the livers and preparation of the RNAs:

6 ten-day-old rats (Wistar) are separated from their mother three hours before the administration of an oral dose of glucose (200 mg/animal). One animal is sacrificed immediately after gavage (T 0 H). The others are sacrificed every hour (T 1 H to T 5 H). The livers are removed and then ground immediately after the animal's death. The RNAs are prepared according to the RNAwiz commercial protocol (Ambion).

### c) Differential display:

The total cDNAs are synthesised by reverse transcription with Omniscript Reverse Transcriptase (Qiagen) from each of the RNA preparations described above. The differential display procedure follows the protocol of the DELTA differential display kit (Clontech). The cDNAs were amplified by PCR by means of the consecutive use of pairs of random primers according to the following PCR conditions: three cycles of 5 minutes at 94°C, 2 minutes at 40°C and 5 minutes at 68°C, then 30 cycles of 45 seconds at 94°C, 1 minute at 60°C and 2 minutes at 68°C, and finally 7 minutes at 68°C. The PCR products generated in this way were separated by electrophoresis on 4.5% acrylamide gel (Genomix). The EIIH cDNA was synthesised using the pair of oligonucleotides P6: 5'-ATTAACCCTCACTAAATGCTGGGTG-3' (SEQ ID No. 5) and T11: 5'-CATTATGCTGAGTGATATCTTTTTTTTTTTG-3' ((SEQ ID No. 6). This DNA was excised from the gel, eluted, reamplified by PCR with the P6 and T11 primers and then sequenced.

### d) Rapid amplification of the cDNA ends (RACE):

To obtain the total cDNA of the EIIH gene, the inventors followed the SMART RACE commercial procedure (Clontech) and then used the primers U6: 5'-ACGCGGGGGGGTCGCCTAGGTG-3' (SEQ ID No. 7) and L10: 5'-GATGGAAAGAGCTCTTACATGTGTTTATT-3' (SEQ ID No. 8), corresponding respectively to the 5' and 3' ends of the cDNA, in order to synthesise and then clone the total cDNA.

### Results

To identify novel hepatic genes regulated by insulin, the inventors used the differential display technique. The preferential study was conducted in parallel on each of the *in vitro* and *in vivo* models described above. The selection criterion applied to the PCR products amplified only in the presence of insulin.

Out of 110 genes, 30 exhibited a differential character. These 30 candidate genes were cloned and the differential character of these genes was then retested by semiquantitative RT-PCR, under different incubation conditions by the insulin in the liver and the hepatocytes in culture. This test made it possible in particular to eliminate the false positives and the known responder genes.

Finally, the pair of primers P6 and T1 generated an 800 bp DNA fragment whose differential profile corresponded to the selection criteria. It was called EIIH. Using this DNA as a probe for northern blotting, the inventors detected a 2.5 kb signal corresponding to the size of the messenger RNA expressed in the liver.

The full length EIIH cDNA was then synthesised by PCR from the sequence obtained by the 5' and 3' RACE experiments.

Computer analysis of this sequence indicates the presence of an ORF (open reading frame) coding for a protein.

To find out whether this protein was translated, the inventors cloned the cDNA into an expression vector, namely pTargeT™ Mammalian Expression System (Promega). *In vitro* transcription/translation experiments (TNT Coupled Reticulocyte Lysate System, Promega) performed on this construction show that the EIIH gene codes for a protein whose apparent size on acrylamide gel is 35 kD (expected theoretical size = 38 kD).

### Example 2:

### Study of the intracellular location of the protein

To find out the intracellular location of this protein, the inventors expressed, in cells, the EIIH protein fused to another protein, namely green fluorescent protein (GFP), which is readily detectable by direct fluorescence in microscopy.

This was done by cloning the coding sequence of the EIIH cDNA into an appropriate vector (pEGFP-N1), making it possible to express the GFP, fused to the C-terminal part of the EIIH protein, in cells previously transfected with this plasmid (transitory expression).

### a)- Transfection of COS cells with the plasmid expressing the EIIH. protein fused to GFP

COS cells are transfected with the above-described plasmid construction using the lipofectamine technique.

Observation by fluorescence microscopy shows that about 40% of the cells are fluorescent. The labelling is intense and seems to be concentrated at certain points in the cells.

Observation of these cells by confocal microscopy makes it possible to refine the visualisation of the cell location of the EIIH-GFP fusion protein. To do this, the inventors reproduced these experiments and plated and then fixed the cells (with paraformaldehyde) on slides, which are visualised by confocal microscopy.

The fluorescence observed in these cells is intense and seems to be concentrated at certain points located in the cytoplasm (no fluorescence observed in the nucleus or on the plasmic membrane). The distribution of this labelling indicates that this protein is not cytosoluble, but rather seems to be associated with cytoplasmic micro-organelles, which can be the endoplasmic reticulum and/or the Golgi apparatus and/or structures of vesicular type (secretion or the like). The inventors noted the constant presence of a more intense fluorescent labelling in a zone close to the nucleus, which might be the Golgi apparatus. The association of this protein with vesicular structures suggests that it could be secreted.

### b)- Study of the intracellular location of the EIIH-GFP fusion protein in CHO-IR cells

To confirm these results, CHO cells constitutively expressing the insulin receptor (CHO-IR) were transfected with the plasmid expressing the EIIH-GFP fusion protein.

Observation of these cells by confocal microscopy indicates that the intracellular location of the EIIH-GFP fusion protein is similar to that observed previously in COS cells.

### Example 3:

### Expression of the EIIH gene in different cell lines

The inventors then studied the expression of the EIIH gene in a tissue (placenta) and cell lines different from those tested previously, namely placenta, FAO, HepG2, H4IIE, AtT20, HIT, MCF-7 and INS-1. According to northern blotting analysis, this gene does not seem to be expressed in HepG2 cells or HIT cells. On the other hand, the gene is expressed in rat placenta, FAO and AtT20 cells, MCF-7 and INS-1 cells and, to a lesser extent, H4IIE cells.

### Example 4:

### Effects of insulin on the expression of the EIIH gene in the rat

The EIIH gene is expressed in the suckled rat liver following the administration of glucose (i.e. after an increase in the insulinaemia) and in primary culture hepatocytes after the addition of insulin to the culture medium.

The inventors used northern blotting to study more precisely the induction kinetics of this gene in the liver. This gave the following information:
- The addition of insulin to primary culture hepatocytes from 12-day-old animals causes a rapid and transitory accumulation of the EIIH messenger RNAs. In fact, the expression of this gene is observed to peak on average three hours after the addition of insulin. This observation is repeated on hepatocytes from (fasted) adult animals, suggesting that this gene plays a role following the addition of insulin, irrespective of the animal's age.
- *In vivo,* in the livers of 12-day-old animals, the EIIH gene is induced in less than one hour after stimulation by insulin.

In addition, twelve adult rats were treated with streptozotocin. 3 days after the injection, 6 rats have glycaemia of between 350 and 500 mg/dl. The liver was then removed from 4 rats (basal condition). The inventors injected the other 2 rats with insulin (20 U/kg) and removed their liver 2 h after the injection. After preparing the RNAs from these different organs, they measured the level of expression of the gene by the PCR technique in real time and compared the expression of the gene before and after treatment with insulin. Injecting STZ rats with insulin causes a 3-fold increase in the expression of the EIIH gene, confirming *in vivo* the stimulating effect of insulin alone on the level of expression of the EIIH gene.

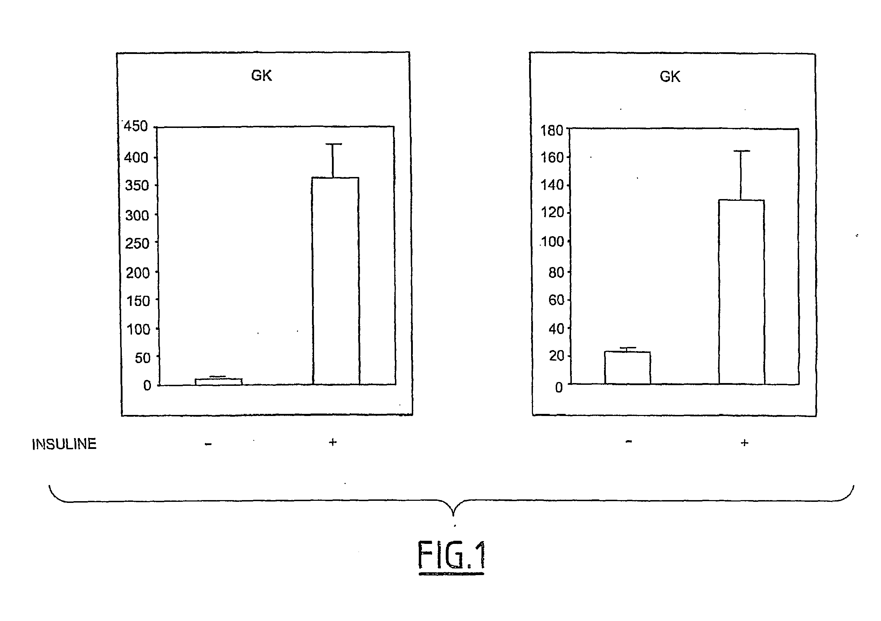

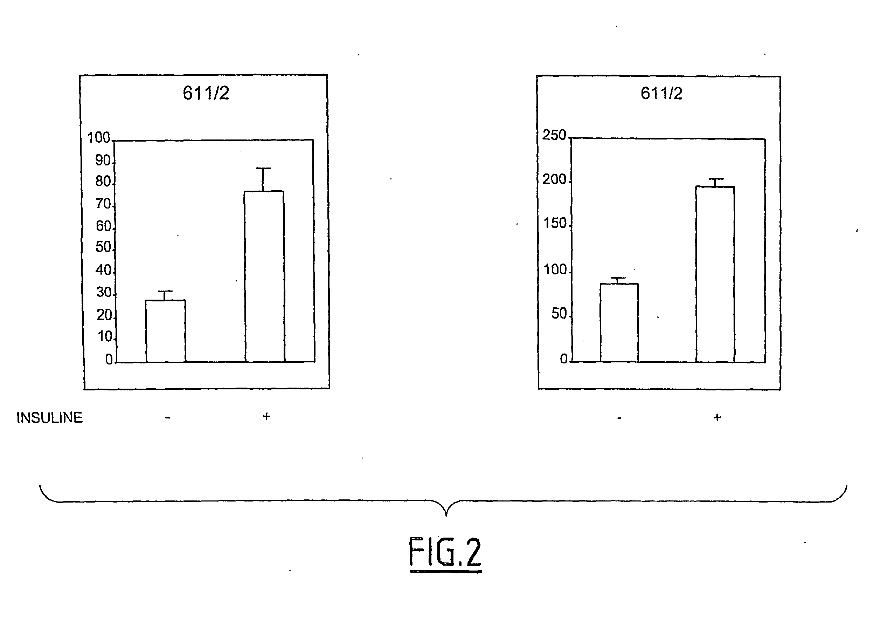

The results of two independent experiments are shown in Figures 1 and 2.

In all the experiments conducted, it was noted that the accumulation kinetics of the EIIH messenger RNA are early and precede those of glucokinase.

### Example 5:

### Expression of the EIIH gene under the effect of insulin in the mouse

The inventors used northern blotting to study the expression and regulation of the EIIH gene by insulin in the mouse. To do this, they administered an oral dose of glucose to previously fasted mice and sacrificed them at the following different times: 0.1 h, 2 h, 4 h, 6 h, 8 h and 10 h. The inventors observed an induction of the EIIH gene over time which was similar to that observed in the rat.

This result reinforces the link between the expression of the EIIH gene and the effect of insulin, since it makes it possible to extend the results obtained in the rat to another animal species, namely the mouse, indicating that this effect is not peculiar to the rat.

### Example 6:

### Reduction of EIIH expression inhibits glucose uptake in the H4IIE hepatocyte cell line

The expression pattern of EIIH in the liver of both newborn and fasted/ fed adult rats and its parallel tissue distribution with glucose transporter 2 (Glut-2) suggests a possible role of EIIH in glucose uptake and metabolism.

A way to test this hypothesis is reduction of the gene expression followed by functional analysis in the pertinent cell systems. During the last few years an effective reduction of gene expression by small interfering RNA, siRNA, has been reported (1-3). This method is well explained in the literature and is now very widely applied to many different cell types.

As the primary assay we measured the glucose uptake of the H4IIE rat hepatic cell line in which EIIH mRNA was reduced to about 70% by specific siRNA. The siRNA was a double-stranded synthetic RNA fragment matching nucleotides 86-105 (on the full length EIIH mRNA) and was applied to H4IIE by means of the transfection reagent jetSI according to the manufacturer's instructions (www.polyplus-transfection.com). Two days after treatment with siRNA, when the EIIH mRNA was reduced to 75% or more (Fig. 3), the glucose uptake was measured at two different concentrations. A moderate but significant and reproducible reduction of glucose uptake was observed at glucose concentrations of 0.2 to 0.8 mM (Table 1). The method of glucose uptake, indicated below, was basically the same as reported previously (4) with minor modifications.

The mechanism by which the reduction of EIIH mRNA, and most probably its protein, leads to the inhibition of glucose uptake remains to be determined, but these preliminary results point to the involvement of this gene in carbohydrate metabolism and can be a potential candidate as a target for diabetes drug development.

### Results: Glucose uptake of the siRNA treated H411E cells

Cells were cultured at 8 x 10⁵/well of 6-well plates overnight. They were then transfected with siRNA-86 at 60 nM and, 48 hours after treatment, they were incubated in Krebs Ringer Buffer for 3 hours. [³H]-2-deoxyglucose (0.1 µCi/ml) was then added to the cells to a final concentration of 0.01, 0.2 or 0.8 mmol/I in the same buffer. The cells were incubated for an additional 15 min at 37°C, washed twice in ice-cold PBS and lysed in 0.3% SDS for 30 min at 37°C. An aliquot was counted for ³H. The control cultures were non-transfected, transfection reagent alone, transfection reagent and mismatch siRNA.

### References:

1 - Montgomery M.K., Xu S., Fire A. RNA as a target of double-stranded RNA-mediated genetic interference in Caenorhabditis elegans Proc. Natl. Acad. Sci., 95, 15502-15507, 1998**.**
2 - Nykanen A., Haley B., Zamore P.D. ATP requirements and small interfering RNA structure in the RNA interference pathway Cell, 107, 309-21, 2001**.**
3 - Tuschl T., Zamore P.D., Lehmann R., Bartel D.P., Sharp P.A. Targeted mRNA degradation by double-stranded RNA in vitro Genes Dev., 13, 3191-7, 1999**.**
4 - Yuan Gao, Ken Walder, Terry Sunderland, Lakshmi Kantham, Helen C. Feng, Melissa Quick, Natalie Bishara, Andrea de Silva, Guy Augert, Janette Tenne-Brown and Gregory R. Collier Elevation in Tanis Expression Alters Glucose Metabolism and Insulin Sensitivity in H4IIE Cells Diabetes, 52, 929-934, 2003**.**

H4IIE cells were transfected with different siRNAs (siRNA 86, siRNA F, siRNA Q, siRNA mismatch sequence of IGF-II(M)) and with the transfectant reagent jetSi (J) alone. Cultured cells were analysed by northern blotting 48 h after incubation.
(nJ): siRNA at 60 nM without transfectant reagent
(NT): non-transfected

**Table 1**

| Glucose transport in H4IIE cell -line | | | | | |
|---|---|---|---|---|---|
| | conditions | nmol incorp./ 15 min/mg prot. | mean nmol/ 15 min/mg prot. | SD | %/controle |
| **[Glucose] 0.2 mM** | **Control** | 11.65 | | | |
| | | 13.48 | **12.488** | 7% | **-** |
| | | 12.33 | | | |
| | **jetSi** | 12.45 | | | |
| | *(Transfectant)* | 12.04 | **11.848** | 6% | **95%** |
| | | 11.06 | | | |
| | **siRNA IGFII Mismatch** | 11.65 | | | |
| | | 11.57 | **11.468** | 2% | **92%** |
| | | 11.18 | | | |
| | **siRNA EIIH-86** | 10.90 | | | |
| | | 9.96 | **10.512** | *5%* | **84%** |
| | | 10.67 | | | |
| **[Glucose] 0.8 mM** | **Control** | 39.33 | | | |
| | | 37.09 | **37.499** | 4% | - |
| | | 36.08 | | | |
| | **jetSi** | 39.79 | **35.464** | 11% | |
| | | 33.53 | | | **95%** |
| | | 33.08 | | | |
| | sIRNA IGFII Mismatch | 41.61 | **37.734** | 9% | **101%** |
| | | 35.87 | | | |
| | | 35.72 | | | |
| | siRNA EIIH-86 | 33.50 | **32.744** | 4% | **87%** |
| | | 33.35 | | | |
| | | 31.38 | | | |

## Claims

1. Pharmaceutical composition comprising the E2IG4 protein of SEQ ID NO. 2 or 4 in association with a pharmaceutically acceptable vehicle.

2. Use of the product of the E21G4 gene of SEQ ID NO. 1 or 3 for the manufacture of a drug for the treatment of diabetes or its complications, obesity or insulin resistance.

3. Pharmaceutical composition comprising a nucleic acid of SEQ ID NO. 1 or 3 coding for the E21G4 protein of SEQ ID NO. 2 or 4, in association with a pharmaceutically acceptable vehicle.

4. Use of a nucleic acid of SEQ ID NO. 1 or 3 coding for the E2IG4 protein of SEQ ID NO. 2 or 4 for the manufacture of a drug for the treatment of diabetes or its complications, obesity or insulin resistance.

## Patentansprüche

1. Pharmazeutische Zusammensetzung, enthaltend das E2IG4-Protein von SEQ ID NO. 2 oder 4 zusammen mit einem pharmazeutisch unbedenklichen Vehikel.

2. Verwendung des Produkts des E21G4-Gens von SEQ ID NO. 1 oder 3 für die Herstellung eines Wirkstoffs für die Behandlung von Diabetes oder seinen Komplikationen, Fettsucht oder Insulinresistenz.

3. Pharmazeutische Zusammensetzung, enthaltend die Nucleinsäure von SEQ ID NO. 1 oder 3, die für das E2IG4-Protein von SEQ ID NO. 2 oder 4 kodiert, zusammen mit einem pharmazeutisch unbedenklichen Vehikel.

4. Verwendung einer Nucleinsäure von SEQ ID NO. 1 oder 3, kodierend für das E21G4-Protein von SEQ ID NO. 2 oder 4 für die Herstellung eines Wirkstoffs für die Behandlung von Diabetes oder seinen Komplikationen, Fettsucht oder Insulinresistenz.

## Revendications

1. Composition pharmaceutique comprenant la protéine E21G4 de SEQ ID NO. 2 ou 4 en association avec un véhicule pharmaceutiquement acceptable.

2. Utilisation du produit du gène E21G4 de SEQ ID NO. 1 ou 3 pour la fabrication d'un médicament destiné au traitement du diabète ou de ses complications, de l'obésité ou de l'insulinorésistance.

3. Composition pharmaceutique comprenant un acide nucléique codant pour la protéine E21G4 de SEQ ID NO. 1 ou 3 en association avec un véhicule pharmaceutiquement acceptable.

4. Utilisation d'un acide nucléique de SEQ ID NO. 1 ou 3 codant pour la protéine E2IG4 de SEQ ID NO. 2 ou 4 pour la fabrication d'un médicament destiné au traitement du diabète ou de ses complications, de l'obésité ou de l'insulinorésistance.
